# EUROPEAN PATENT SPECIFICATION

(11) **EP 0 438 520 B1**
(45) Date of publication and mention of the grant of the patent: **24.08.1994**
(21) Application number: 89912306.1
(22) Date of filing: 10.10.1989
(51) Int. Cl.: B03C 1/02, G01N 33/543, G01N 33/50

(54) **SYSTEM FOR MAGNETIC AFFINITY CELL SEPARATION FROM CELL CONCENTRATES**
SYSTEM ZUR MAGNETISCHEN AFFINITÄTS-SEPARATION VON ZELLEN AUS ZELLKONZENTRATEN
SYSTEME SERVANT A SEPARER PAR AFFINITE MAGNETIQUE DES CELLULES D'UN CONCENTRE CELLULAIRE

(30) Priority: 11.10.1988 US 255214; 22.08.1989 US 397067
(43) Date of publication of application: 31.07.1991
(73) Proprietor: BAXTER INTERNATIONAL INC., Deerfield, IL 60015 (US)
(72) Inventor: HARDWICK, R., Alan, El Toro, CA 92630 (US); LAKE, William, C., Laguna Niguel, CA 92656 (US); PRISCO, Michael, R., Aurora, IL 60505 (US)
(74) Representative: MacGregor, Gordon
(86) International application number: US8904527
(87) International publication number: WO9004019

(56) References cited:
- EP-A- 0 184 710
- WO-A-83/02405
- WO-A-87/06844
- WO-A-88/06632
- WO-A-89/06280
- US-A- 4 710 472

## Description

### TECHNICAL FIELD

The present invention concerns a novel apparatus and method for separating a specific cell population from a heterogeneous cell mixture.

### BACKGROUND OF THE INVENTION

In the field of cell separation, it is common to separate cells from plasma in blood and also to separate by centrifugation various types of cells such as red cells from white cells and the like. However, there is often a need to separate cells which are only slightly different from the other cells in a suspension thereof. If the cells are of nearly equal specific gravity, they may not be separated by centrifugation.

For example, it may be desirable to isolate various types of leukocytes from a bone marrow concentrate or a peripheral blood stem cell concentrate. It may be desirable to perform selective separation of neuroblastoma cells from a bone marrow concentrate. It may be desirable to selectively separate specific T-lymphocyte subset populations (helper-inducer or suppressor-cytotoxic T-lymphocytes) from a lymphocyte concentrate that is prepared using a blood cell separator.

Additionally, it may be desirable to selectively separate precursors of lymphokine activated killer (LAK) cells, tumor infiltration lymphocyte (TIL) cells, or activated killer monocytes, from lymphocyte or monocyte cell concentrates or from a tissue cell preparation.

US-A-4710472 discloses a device for removing magnetic bead-coated cells from a heterogeneous cell mixture using magnetic means.

Magnetic separations of individual subsets of cells from larger populations in significant quantities become possible. This, in turn, opens up new vistas of research and therapeutic techniques, making use of the purified cell populations which may be obtained.

The pre-characterising parts of claims 1 and 20 contain features present in the disclosure of US-A-4710472, and the distinguishing features of the present invention are set out in the characterising parts of claims 1 and 20.

Another current practice in the field for cell separation utilizes hollow fiber, flat sheet membrane or packed bed bead or particle matrix materials with physically adsorbed or covalently attached chemicals or biochemicals for the selective cell separation from whole blood or the like. These devices are designed to allow continuous whole blood or blood component inflow and return. Since these devices operate at normal blood flow rates under conditions in which the concentration of desired cells can be very low compared with other cell types, the separation process is often not efficient.

In this invention, an apparatus and a method are provided for selective separation of a specific cell population from a heterogeneous cell mixture. In a preferred embodiment, the heterogenous cell mixture may first be subjected to a means for obtaining a selective cell concentrate from the heterogeneous cell mixture by separating the selective cell concentrate based upon the physical properties of the concentrate. The selective cell concentrate is used with the apparatus and method embodying the invention. Specifically, centrifugation may be used as a first separation step. There are many different types of cell centrifuge systems for the separation of desired types of cells.

In the preferred embodiment, a flexible, collapsible, aseptically sealed container is provided, having particle means inside, with the particle means preferably having chemically covalently attached thereto a substance capable of binding to the desired cells only, to the exclusion of other cells. Examples of such a covalently bonded substance may include antibodies, antigens, proteins, glycoproteins, polysaccharides, or lipopolysaccharides. The particle means to be in long term contact with the container walls or the like.

Means are provided for intimately contacting either the heterogenous cell mixture or the cell concentrate with the particle means within the container. This may simply be a flexible, aseptic conduit system which communicates between the site of formation of the cell mixture orconcentrate and the container which has the particle means. Incubation of the cell mixture or concentrate with the particle means may then be permitted, to cause selective binding of a specific cell population from the cell concentrate to the particles, creating a particle/cell conjugate.

Then, since the new particle/cell conjugate will have significantly different properties from the remainder of the cells, it becomes an easy matter to separate them from other cells, by centrifugation, for example. Preferably, one may use particles which are paramagnetic, then effecting separation using magnetic members.

The initial cell fluid volume may remain within the container during the incubation period, when binding to the particles takes place. Also, means may be provided for introducing additional volume of the cell mixture or concentrate into the container during the incubation period.

After separation of the particle/cell conjugate from other cells, as described above, one may separate the specific cell population from the particles or vice versa, for example by eliminating the bond between the particles and the cells in known manner, so that a purified, selected population of cells may be provided for further use. Alternatively, the unbound cells may be the desired cells, being removed from the particle/cell conjugates.

When the particles are paramagnetic, the particle/cell conjugate may be retained in a fixed location by action of the magnetic members, as remaining, unbound portions of the cell concentrate are removed from the location.

The magnetic members which retain the particle/cell conjugate define first and second spaced magnetic members, one being downstream of the other, so that the second, downstream, spaced magnet member can pick up any bound particle/cell conjugate that is lost by the first magnet member, so that no particle/cell conjugate goes downstream with remaining cells.

The magnetic members may be positioned adjacent to means for carrying and positioning the flexible, collapsible container which carries the particle/cell conjugate, to permit at least one inner wall portion of the container to be within the magnetic field of a magnetic member. Thus, this inner wall portion serves as the fixed location at which the particle/cell conjugate is retained.

Flat-pressing means may also be provided, to press the collapsible container flat while the inner wall portion is within the magnetic field, to facilitate separation of the particle/cell conjugate from the remainder of the cell concentrate.

Specifically, the second magnetic member possesses a magnetic field that is shallower (ie, less extensive in distance) than the magnetic field of the first magnetic member, but stronger adjacent the magnet surface for stronger paramagnetic particle retention. Preferably, the magnetic reach of the first magnetic member is substantially equivalent to the width of the fluid container being placed thereon. This ensures that the majority of the paramagnetic particles in the container will fall within the reach of the magnetic field and be drawn to the magnet surface.

It is also preferred for the container which contains the particle means to be aseptically connected to a flexible, multiple-chamber insert member for a blood cell separation centrifuge. Such an insert member may be the disposable, blood-carrying, inner, flexible portion typically used in such a centrifuge. It may be integrally aseptically connected to the container, so that freshly collected blood cells may be aseptically transferred from the insert member to the container having the particle means, without any need of forming a sterile connection therebetween. This greatly simplifies the use, and also increases the likelihood that there is not breach of aseptic conditions.

Also, a first container having the particle means may be aseptically connected to further flexible, collapsible container means, for receiving processed cells from the first container which contains the paramagnetic particle means, or particle means of another type, if desired. Typically, a second flexible, collapsible container is sealingly, aseptically connected to and positioned between the first container described above and the further collapsible container means. This may serve as a downstream catch area which is positioned against the second magnetic member described above to catch any particle/cell conjugate which escapes the first magnetic member against which the first container may be pressed during the cell separation operation. The second flexible, collapsible container may preferably be of hexagonal shape with inlet and outlet ports at opposed corners, to cause generally slow flow of cells through said second container relative to flow lines connected thereto. The flow line diameter is typically no more than one-fourth the width of the second container. Also the flow path, particularly through the second container during magnetic separation, should be of a very shallow depth, typically 0.508 mm to 2.54 mm (0.02 to 0.1 inch).

Further in accordance with a preferred embodiment, one may practice a method which includes the following steps. Blood from the patient may either be collected in a first container or the patient may be connected to a blood separation centrifuge, the centrifuge being operated to form a cell concentrate which is collected in the first container. The first container is sealed. If the particle means are not already in the container, they may be placed in the container in some aseptic manner, or an inner container positioned within the container may be broken from outside of the container to cause their release within the container. Thus the cells and the particle means are mixed. Then, the primary container is connected, if not already integrally connected, to the inlet of a clamped separation set. The primary container and a desired secondary chamber between the primary container and the rest of the separation set are both placed in magnetic separator means. A primary magnet attracts the particles of the particle means, which by now are bonded to the desired cells, to retain the particle means in the primary bag as the remaining contents of the bag flow toward the separation set. The same principle takes place in the secondary chamber under the influence of a secondary magnet, to eliminate or greatly reduce the possibility of any of the particle means and attached cells flowing downstream with the rest of the contents of the primary container.

A clamp is then opened and/or a pump actuated to initiate flow from the primary container through the secondary container and across the magnetic field of the secondary magnet, into storage containers of the separation set. The storage containers may then be sealed, followed by optional disconnection of the storage containers.

Then the magnets may be removed and the adhering cells flushed, so that one or more storage containers may contain pure cell/particle conjugate, while other storage containers contain the remaining contents of the primary container. After this, a conventional process may be used to separate the particles of the particle means from their attached cells, with the cells then being separated by centrifugation or other desired means, such as filtration or magnetic separation, and sent to a container for storage of the pure cells. The means used to break the connection between the particles and the cells depends, of course, upon the specific bonding agent. For example, the particle means may be coated with a antibody for the specific desired cells, with the result that the cells bond to the particle means. Then, when the bond is to be broken, an appropriate reagent may be used to break the bond.

An advantage of the method is use of the magnetic separating apparatus with a cell concentrate in a batch process. In the cell concentrate the specific cell population to be separated is present at higher concentration, which tends to favor separation kinetics. Another advantage is that numerous unwanted blood cell types, in the situation where blood cells are being separated, may have already been greatly reduced in number by the preliminary, typically centrifugal cell separation process, to reduce non-specific cell reactions. For example, the collection of a lymphocyte cell concentration with minimal red blood cell, platelet, and granulocyte contamination may be effected using a blood cell separator.

Additionally, introduction of the particles of the particle means to a cell concentrate under the conditions described above allows incubation to take place at constant volume conditions under storage within the container having the particle means. Thus, the incubation solution composition can be configured to more easily obtain favorable final incubation conditions for formation of the particle/cell conjugate. These conditions may then be optimized for a specific purpose in a way which is far more versatile than otherwise.

The subsequent separation of the particle/cell conjugate is also advantageously achieved, with separation times being faster, and fewer cell types in the original concentrate providing a final product with fewer non-desired contaminating cells.

As previously stated, the material which is covalently attached to the particle means may be, for example, an antibody, antigen, protein, glycoprotein, polysaccharide, lipopolysaccharide. The material may also be a nucleic acid, a lipid molecule, or a synthetic or chemically modified component of such a substance which shows a selective binding affinity for the cell population to be separated. The methods used for the chemical covalent attachment of such are known and used in the production of coupled matrix material for affinity chromatography and other selective adsorption applications. Examples of such techniques of covalent attachment to sepharose, gelating, or other beads may be seen from the following articles: Habeeb, "A Novel Preparation of Immunoadsorbents," Biochimica et Biophysica Acta, 673 (1981) 527-538; Cambier, et al., "Isolated Phosphorylcholine Binding Lymphocytes. I. Use of a Cleavable Crosslinking Reagent For Solid-Phase Adsorbent Isolation of Functional Antigen Binding Cells," Journal of Immunological Methods, 51 (1982) 209-221; and Bonnafous, et al., "Ligands Immobilized Through Cleavable Mercury-Sulfur Bonds.: Journal of Immunological Methods, 58 (1983) 93-107.

The solution in which the particle means may be suspended can be a buffered sale solution which may contain a protein such as albumin, and is compatible with the physiological requirements of the heterogeneous cell concentrate and the biological binding material attached to the particle means. This solution may be configured in its chemical composition and properties to confer sterility to the substance which is covalently attached to the bead or particle. Furthermore, the solution may be configured in its chemical composition and properties such that when a bead or particle suspension is added to the heterogeneous cell concentrate, the properties of the resulting mixture favor the formation of the bead or particle conjugate.

For example, the heterogeneous cell mixture or concentrate may be a bone marrow preparation in which the cells may be further concentrated in a cell concentrating centrifuge or the like. Additionally, the heterogeneous cell mixture can be a tissue derived cell suspension, or a cell concentrate prepared from peripheral blood using such centrifugal device. Examples of the latter are concentrates of platelets, lymphocytes, granulocyte, monocytes, or peripheral bone marrow stem cell preparations prepared with a blood cell separator such as the previously described CS3000 blood cell separator or Autopheresis-C device.

The beads or particles used in the system may be selected for particular size and specific gravity properties so as to allow the subsequent separation of the beads or particle/cel conjugate from the unconjugated cells in the heterogeneous cell concentrate using the centrifugal capabilities of a blood cell separator. A solution such as Ficoll-Hypaque or Percoll might be used to facilitate this separation.

The beads or particles of the particle means may be composed of any number of different materials such as polystyrene, latex, plastic copolymers, glass, synthetically produced gel beads and the like. Preferably, such materials will possess good mechanical properties to prevent flaking or fracturing of the beads or particles, and will allow chemical covalent attachment with ease.

It is further preferred for the beads or particles to be formed around a magnetite particle, for example, to allow separation of the bead or particle/cell conjugate using magnets, as described above. For example, particles may be produced in accordance with the methods as described in EP-A-0344270

The typical size of the particle means used in this invention may be from about 2 to 10 microns, preferably about 3 to 5 microns. The particles may be added in a liquid suspension, forming a typically dark sludge-like material.

On the order of 10 ml. of such liquid suspension may be placed in the bag which is to receive the cells for separation typically including one hundred thousand to 20 billion particles. In the event it is undesirable for any reason for the particles to remain in the bag for an excessively long time, due, for example, to interaction with the bag wall, they may be added separately to the bag by conventional means such as using a sterile connector, or they may reside in a frangible container within the bag, to be broken when their use is desired so that they enter the bags interior from the frangible container.

As stated above, the various containers used in this application are preferably integrally linked together in their initial manufacture so as to avoid the need for sterile connection during the processing. However, they may also be connected together with sterile connectors, numerous designs of which are well-known, for example, those of U.S. Patent No. Re. 32,056.

The Dynal Company of Great Neck, New York manufactures paramagnetic microbeads which may be used in accordance with this invention.

For a typical blood cell separation, the number of microbeads of the particle means used may number from about a hundred thousand to one billion. It has been found, when making use of a primary and secondary magnetic separator as described above, that the removal of microbeads and the cells attached to them from a cell suspension may be quantitative, with virtually no microbeads found in downstream effluent after passage through the magnetic separation apparatus.

### DESCRIPTION OF THE DRAWINGS

Figure 1 is a partially schematic plan view of apparatus for selectively separating cells.

Figure 2 is a perspective view of part of a magnetic separation device used with the apparatus of Figure 1.

Figure 3 is a perspective view of another part of magnetic separator used with the apparatus of Figure 1.

Figure 4 is a perspective view of the separation system in operating position.

Figure 5 is a plan view of a compound magnet used in the magnetic separator of Figures 2-4.

Figure 6 is an end view of the magnet of Figure 5.

Figure 7 is an end view of a second compound magnet used in the magnetic separator of Figures 2-4.

### DESCRIPTION OF THE SPECIFIC EMBODIMENTS

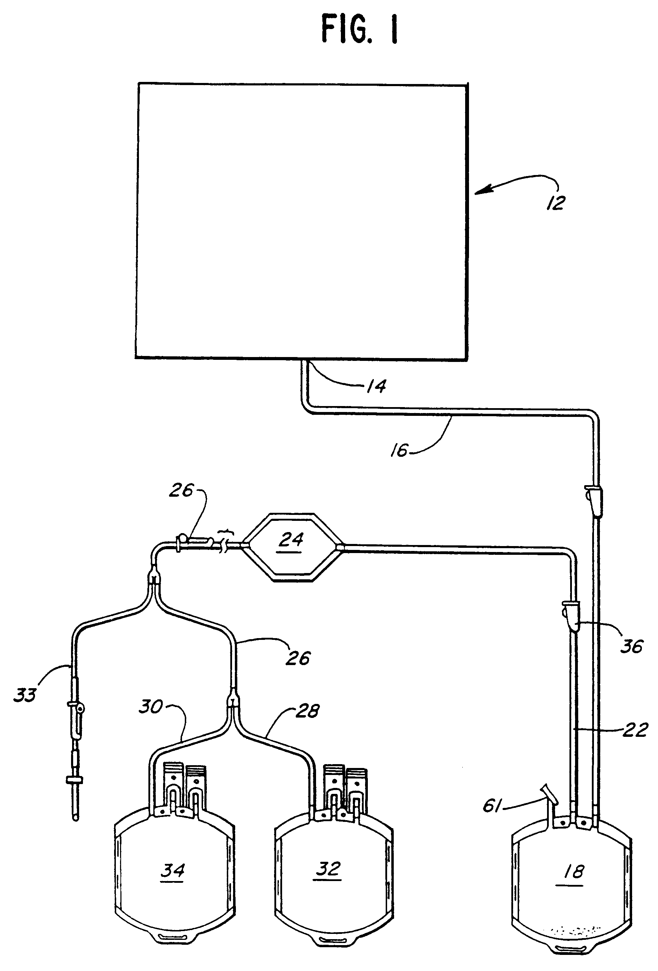

Referring to Figure 1, a disposable system 10 is disclosed for separating an individual population or populations of cells from a heterogeneous cell mixture.

Cell concentrator portion 12 which may be used withthis invention is schematically shown and may be of a design as shown in U.S. Patent Nos. 4,379,452 or 4,410,026, or may be any cell concentrator member usable in any known apparatus for the concentrating of cells. For example, the CS3000^{T.M.} separation system sold by Baxter Healthcare Corporation may be used, or the Autopheresis-C^{T.M.} separation system, sold also by Baxter Healthcare Corporation, or any other desired, similar device, may be used.

At outlet port 14 of concentrator system 12, a desired population of cells may be provided, for example lymphocytes, separated in concentrator system 12 from whole blood. The lymphocytes, perhaps mixed with other white blood cells, pass through flexible conduit 16 into flexible, collapsible container 18 of generally conventional design, which contains the particle means of micron-sized particles including a paramagnetic material such as ferric oxide, coated with plastic such as polymethyl methacrylate, which, in turn, is further coated with an antibody or other cell bonding agent to the specific marker proteins in the cell wall of a given category of leukocytes, so that the leukocytes and particles 20 selectively bond to each other, to the exclusion of the remaining leukocytes and other cells.

The apparatus of this invention also carries flexible tubing 22 which communicates between flexible, collapsible container 18 and a flexible collapsible container 24 communicating with tubing 22 at one end and communicating with an outlet tubing 26 at this other end. Tubing 26 branches into tubings 28, 30 each of which connects to another flexible, collapsible container 32, 34.

While cell concentrator portion 12 is preferably integrally connected to flexible container 18, the connection may be made by a conventional sterile connector system, if desired. Additionally, the particles 20 may be stored either outside of container 18 and connected with a sterile connector system, or they may be placed in a frangible container inside of bag 18 with the container being breakable on use so that the particles 20 do not have an excessive amount of time to interact with the bag walls, in the event that some adhesion may take place there. Of course, the particles 20 may reside freely within the bag if the particles do not interact with the bag wall.

After the cells have been separated and concentrated in separation apparatus 12 and conveyed to bag 18, the primary, cell-containing container 18 may be sealed by heat sealing of line 16 and by operation of clamp 36 (although clamp 36 may be closed at an earlier stage of the operation). If the microbeads or particles 20 are not already added to the container, they may be so added at this time, followed by gentle mixing of the contents of the primary container: specifically, the cells and the microbeads or particles. At this time, bonding takes place between the particular cells selected and the antibody coating of the particles or microbeads 20.

Following this, if container 18 is not integrally connected, as shown, with the downstream set portion of this invention beginning with chamber 24 and including bags 32, 34, such connection may be made in sterile, aseptic manner by conventional means, for example, by use of a sterile connector system of known design. In the preferred embodiment shown in Figure 1, the various components are integral to avoid the inconvenience of connection and the risk of contamination of the container contents.

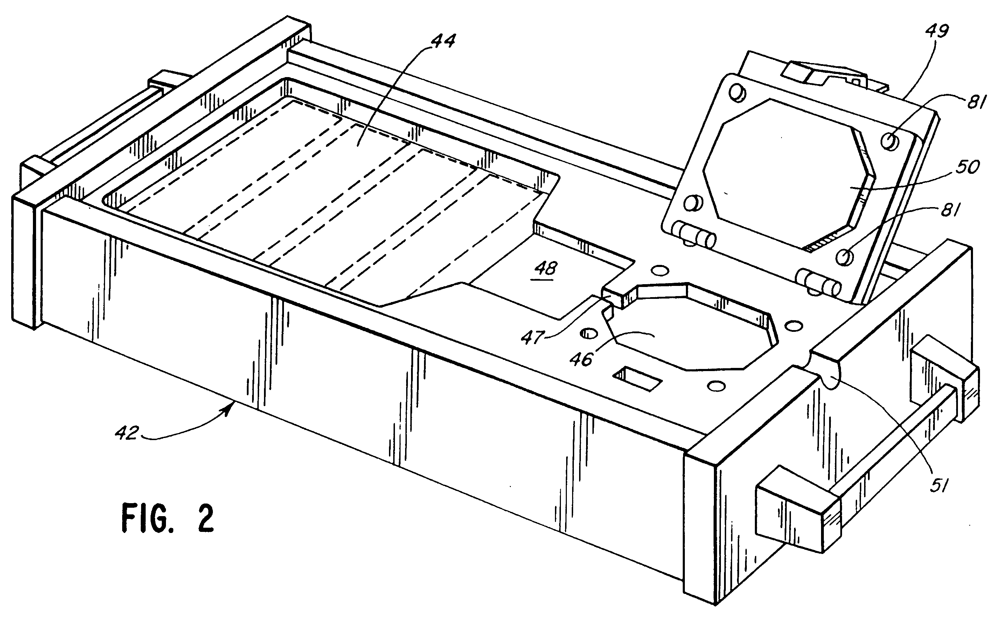

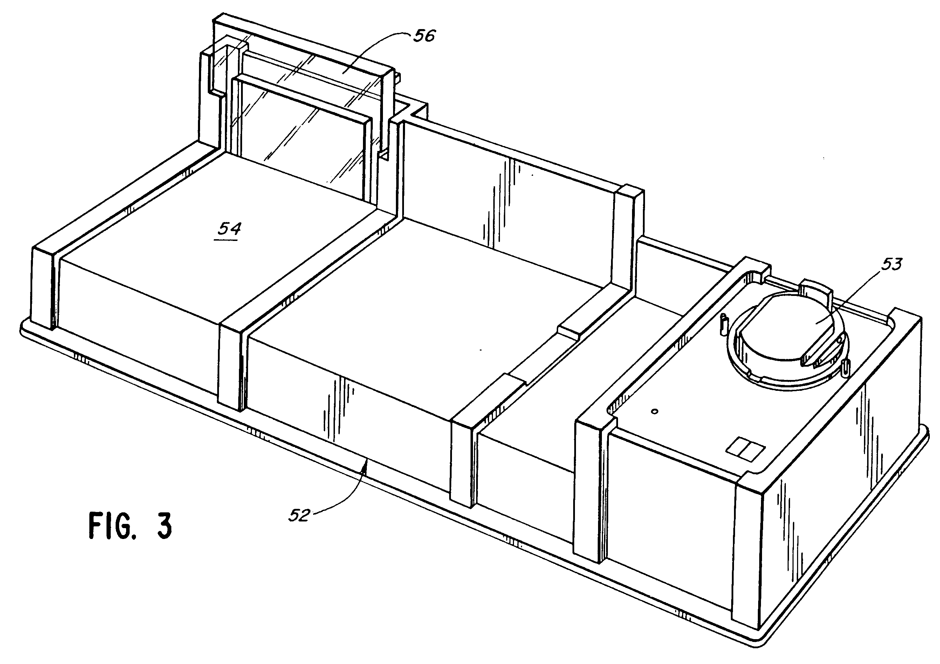

Following this, containers 18 and 24 are placed into magnetic separator 40, which is shown in various aspects in Figures 2-7.

Separator 40 carries upper base member 42 which carries magnet assemblies 44, 46. Upper rotatable, hinged presser member 49 comprises a flat undersurface portion 50, and carried by base 42, overlying in its closed position magnet 46. Upper base member 42 thus provides two spaces, respectively against magnet 44 and magnet 46, which are respectively proportioned to receive bag 18 and bag 24. Channel 47 and space 48 provide room for tubing 22, while channel 51 is provided to receive tubing 26.

Additionally, lower base member 52 is proportioned to receive upper base member 42 in cradle area 54 so that tubing 26 can be connected to roller pump assembly 53 for controlled pumping flow of cells out of bag 18, through intermediate bag 24, and into one or the other of containers 32 or 34.

Hinged cover 56 is positioned to be brought down on top of bag 18 as installed in upper base member 42. Upper rotatable hinged presser member 49 presses down in similar manner on flexible chamber 24. The purpose of particularly presser member 49 is to provide precise definition of the thickness of the flow path of chamber 24 during processing to cause the flow path across the associated magnet 46 to be of very shallow depth, for example, about 1.27 mm (0.05 inch). Similarly, magnet 44 is positioned to associate with chamber 18. As will be described more fully herein, the magnets 44 and 46 are designed to provide suitably configured magnet fields to capture and retain paramagnetic particles passing through the containers positioned thereon, respectively containers 18 and 24. In this regard magnetic 44 will possess a greatermagnetic field reach than magnetic 46 in order to capture a larger percentage of particles. This places the cells passing therethrough into a position to be strongly influenced by the fields generated by magnets 44, 46. Thus, those cells bonded to magnetic beads will be retained adjacent one or the other of the magnets.

One advantage for using a magnetic separator apparatus 40 having a separable upper base member 42 is that upper base member 42 may be refrigerated to a temperature on the order of 4° C. prior to use. Thus, the cold magnets keep the cells cold during operation, as well as providing some increase in magnetic field strength. The cold cells are less active and better preserved. Also, at low temperatures, non-specific cell interactions with the paramagnetic beads can be reduced. For example, phagocytes present are less active in ingesting available beads when kept at low temperatures.

When containers 18, 24 are lying on magnets 44, 46, clamp 36 may be opened, and pivotable pressure member 56 may be gently closed to press the container 18 flat and to press the cells and their carrier liquids out of bag 18, which rests upon magnet 44. Presser 56 may also contain magnetizable metal strips that are sized and positioned so that they are magnetically pulled to the magnet 44 in a way such that the plastic bag 18 is squeezed and the efficient flow from the bag is controlled and relatively constant. Alternatively, the roller pump 53 may be activated to cause the cells and their carrier liquids to flow out of bag 18. As the cells and their carrier liquids flow through tubing 22, the magnetic field from magnet 44 attracts the particles or microbeads 20, and the cells to which they are bonded, causing such microbeads 20 to be generally firmly affixed against the inner wall 57 (Fig. 6) of bag 18 that is closest to magnet 44, holding the particles 20 and their attached cells as the remaining cells and suspension liquid pass through tubing 22 out of bag 18.

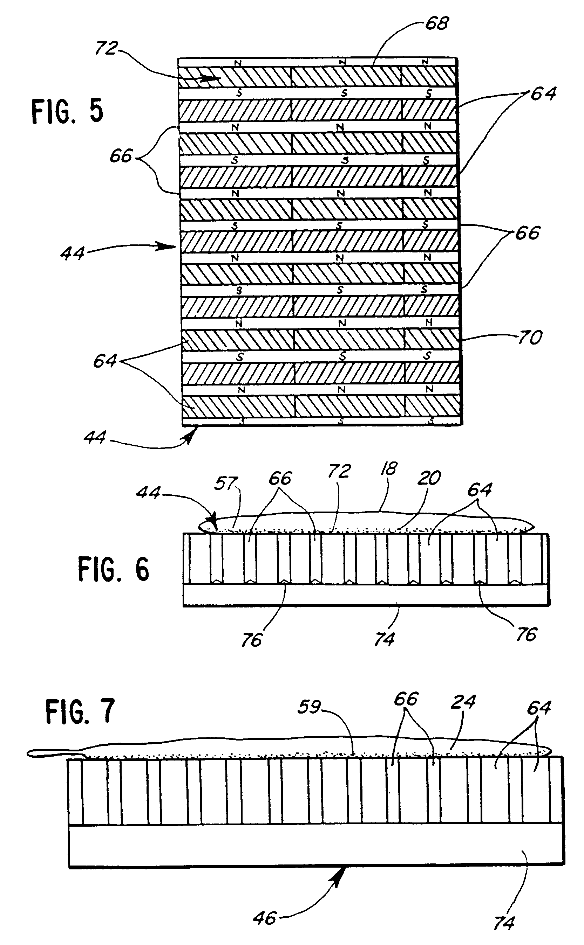

If any particles 20 and attached cells escape the first magnet 44, they may be caught by the interaction between container 24 and second magnet 46, upon which it lies, being retained against inner wall 59 (Fig. 7). Even under significant flow conditions, the enlarged container 24 exhibits relatively slow flow conditions through it. Hinged presser member 49 contains four metal bolts 81 that are sized to hold the presser member 49 in place with the proper spacing and mount of magnetic force. The surface 50 of presser member 49 is precisely machined to precisely define the thickness of the flow path through container 24. The shallow depth of this flow path causes any particles or microbeads present to drift into the influence of the magnetic field of magnet 46, to be retained on the inner wall 59 of container 24 as the remaining cells and liquid flow by, out of container 24, through tubing 26, into bag 32, for example. In such a circumstance, tubing 30 may be clamped off to keep bag 34 empty.

Then, when pressure member 56 has been used to squeeze all possible liquid and cells out of bag 18, a small amount of cell-compatible suspension liquid may be passed through the system, via sterile connector port 61, priming line 33, or another integrally connected container, to flush the remaining cells which are unattached to paramagnetic particles through the system into bag 32.

Then, tubing 28 may be closed, and tubing 30 opened by conventional clamp means. The system may be removed from the field of influence of magnets 44, 46, and, more suspension/solution may be passed into container 18, to flush the cells which are bound to particles 20 through tubing 22, through chamber 24, picking up any bound cells retained there, and into container 34.

Then, containers 32, 34 may be separated from the system by sealing and severing of tubings 28, 30 in conventional manner, with the desired particular population of cells being separated out from the main body of cells, and placed in separate container 34 for separate use.

As stated above, magnet 44 is designed to possess a greater magnetic field reach than magnet 46. Preferably, this magnetic field reach is at least equal to about three-quarters of the width of the container 18, and more preferably substantially equivalent to such width. This ensures that a majority of the paramagnetic particles within container 18 are captured by the magnetic field and drawn to the surface of magnet 44. Accordingly, magnet 44 will have a magnetic field reach of from about 12.7 to 25.4 mm (one-half to one inch), preferably, 12.7 to 19.05 mm (one-half to three-quaters of an inch).

Typically, magnets having greater magnetic field reaches possess lower surface field strengths. One particle magnetic assembly which provides the desired magnetic field reach, but retains substantial surface field strength will be described with reference to Figures 5 and 6. Magnet assembly 46 may be made of similar construction. Magnet assembly 44 is shown to comprise a stack of bar magnets 64 which are separated by, and in contact with, steel pole pieces 66. As a particular advantageous feature, the like poles of adjacent bar magnets 64 in the stack face each other and particular pole piece separating them. This is demonstrated by the letters "N" and "S", each of which indicate the combined north poles or south poles of the respective magnets which are facing each other. The bar magnets 64 define long sides 68 and ends 70, and north and south poles of the bar magnets being defined along an opposed pair of long sides as shown in Figure 5. Preferably, the bar magnets are made of a high magnetism alloy of neodymium, iron and boron. In Figure 5, the face 72 of the magnet assembly shown rests in use against an outer wall of bag 18 so that the magnetic field from magnet assembly 44 passes into container 18, for retention of particles 20.

Turning to Figure 6, an end view of magnet assembly 44 is shown, with face 72 being the face that is displayed in Figure 5. As shown, magnets 64, separated by pole pieces 66, rest upon a non-magnetizable aluminum plate 74 or the like, to support the respective magnets and pole pieces.

It can also be seen that pole pieces 66 each define an angled groove 76 along parallel end faces which are spaced from bar magnets 64, and which are opposed to face 72 of the assembly 44, which face is the fixed location for retaining the particle/cell conjugate formed through the paramagnetic particles as in this invention.

The bar magnets 64, pole pieces 66, and support plate 74 may be bonded together in any conventional manner with non-magnetic cement, or making use of appropriate clamps or retention straps. Appropriate magnets for use herein may be obtained from the Crucible Magnetics Co. of Elizabethtown, Kentucky. Magnet assembly 44 contains magnets 64 that are 12.7 mm (.50 inch) thick and pole pieces 66 that are 6.35 mm (.25 inch) thick. This generates a magnetic field that has local maxima of 7000 to 9100 gauss (average 8300 gauss) at the surface of the magnet and which decreases to 1100 to 1700 gauss (average 1400 gauss) at a distance of 1 cm. from the magnet surface. This magnet assembly gives both a relatively strong magnetic holding force at the magnet surface and a relatively good magnetic "reach out" force to capture beads some distance (up to 25.4 mm (1")) from the magnet surface.

Magnet assembly 46 contains magnets 64 that are 6.35 mm (.25 inch) thick and pole pieces 66 that are 2.54 mm (0.1 inch) thick. This generates a magnetic field that has local maxima at the magnet surface ranging from 7300 to 8000 gauss and which decreases to 80 to 500 gauss at a distance of 1 cm from the magnet surface. Compared to magnet assembly 44, magnet assembly 46 has a stronger magnetic holding force at the magnet surface, but has less magnetic "reach out" force and thus has a shallower magnetic field. The second flexible container 24 is designed to take advantage of the particular magnetic field of magnet 46. First container 18 is shown to rest on magnet assembly 44 for cell separation.

Accordingly, the system isolates in an aseptic, simplified manner, a particular subclass of concentrated cells, which may be separated as shown for any desired purpose.

### EXAMPLE 1

This example illustrates a specific application of the apparatus and method of this invention in the separation of T-helper/inducer lymphocyte cells collected from whole blood.

### Preparation of Mononuclear Cell Suspension:

Approximately 500 ml. of whole blood was collected into a standard Fenwal Sodium Citrate Double Blood Pack and divided between the two packs to give 290 ml. in each pack. Hespan Hetastarch (55 ml.) was added to each pack, and the contents mixed by gently tilting of the pack in a back and forth motion. The red cells were allowed to settle, and the plasma layer which contains mononuclear cells was transferred to a standard Fenwal 600 ml. transfer pack using a Fenwal plasma extractor and standard transfer tubing set. The transfer pack was centrifued to pellet the cells from the plasma, and the plasma was transferred to another transfer pack using the plasma extractor. The cells were resuspended by adding 80 ml. of Hanks Balanced Salt Solution (HBSS) which 10 percent Fetal Calf Serum and gentle titling of the bag back and forth. An aliquot of the cell suspension was counted in 2 percent acetic acid and used to calculate a total cell harvest of 2.8 x 10⁹ mononuclear cells for the cell suspension. After counting of the cells, an additional 100 ml. of HBSS was added to the transfer pack, and the cell suspension was mixed. One-half of the cell suspension was transferred to a second transfer pack to give a total of about 1.4 x 10⁹ mononuclear cells in 100 ml. HBSS in each bag.

### Preparation of Paramagnetic Beads Bonded With Antibody

About 1 gram of paramagnetic beads (Pandex beads with amino surface functional groups) were washed five times with saline solution and resuspended in 20 ml. of saline in a 50 ml. glass test tube. Freshly prepared N-succinimidyl-3-(2-pyridyldithio) propionate (SPDP) comprising 1.0 ml. of 20 millimolar solution of SPDP and absolute ethanol, was added to the test tube. The tube was rotated end-over-end for 30 minutes at 4° C. to form 3-(2-pyridyldithio)-propionyl-derivitized beads. The beads were collected by magnet and washed five times with 20 ml. aliquots of phosphate buffered saline. The beads were then suspended in 20 ml. of 50 millimolar dithiothreitol in sodium acetate (0.1 Ml buffer, pH 4.5) and incubated with end-over-end rotation for 30 minutes at 4° C. to form the thiol derivative. The bead preparation was then washed five times in phosphate buffered saline, collecting the beads with a magnet after each wash. Finally, the beads were resuspended in 20 ml. of phosphate buffered saline.

Mouse anti-Leu 3a antibody (type CD4 antibody-2mg. in 2 ml. phosphate buffered saline) was added to 0.1 ml. of 20mM SPDP in absolute ethanol. The mixture was dialyzed overnight against 500 ml. of phosphate buffered saline.

The resulting antibody solution was added to the derivitized bead suspension in a 50 ml. glass centrifuge tube. The tube was rotated end-over-end overnight at room temperature to cause coupling of the antibody to the beads. Then, the beads were washed five times with phosphate buffered saline and resuspended in 30 ml. of phosphate buffered saline.

Thus, while the created antibody carries pyridyldithio active groups, the beads, because of their dithiothreitol treatment, carry bound sulfhydryl active groups. Accordingly, upon being brought together, the antibody and the beads become chemically bonded together through a disulfide linkage resulting from a condensation reaction, with 2-thiolpyridine being split off as a by-product.

### Coupling of Mononuclear Cells to CD4 Antibody Bonded To Beads

Approximately 300 mg. of the antibody-bonded beads prepared as above (7 x 10⁹ beads) were added to each bag of cell suspension prepared as previously described, using a 20 ml. syringe with attached 21 gauge needle. The resulting suspension had about a 5 to 1 bead to cell ration, and was gently mixed, being then placed on a Cole-Parmer rotator at a setting of 4 for 30 minutes at 4° C. The bag 18 (Fig. 1) containing the bead and cell suspension was connected to a 600 ml. Fenwal transfer pack 32 through tubing 22, 26 which communicates through an enlarge chamber 24, as also illustrated in Fig. 1. Bag 18 was also connected through the priming line 33 of the tubing set to a 1,000 ml. bag of physiological saline solution to allow priming of the set between container 18 and the empty transfer pack 32.

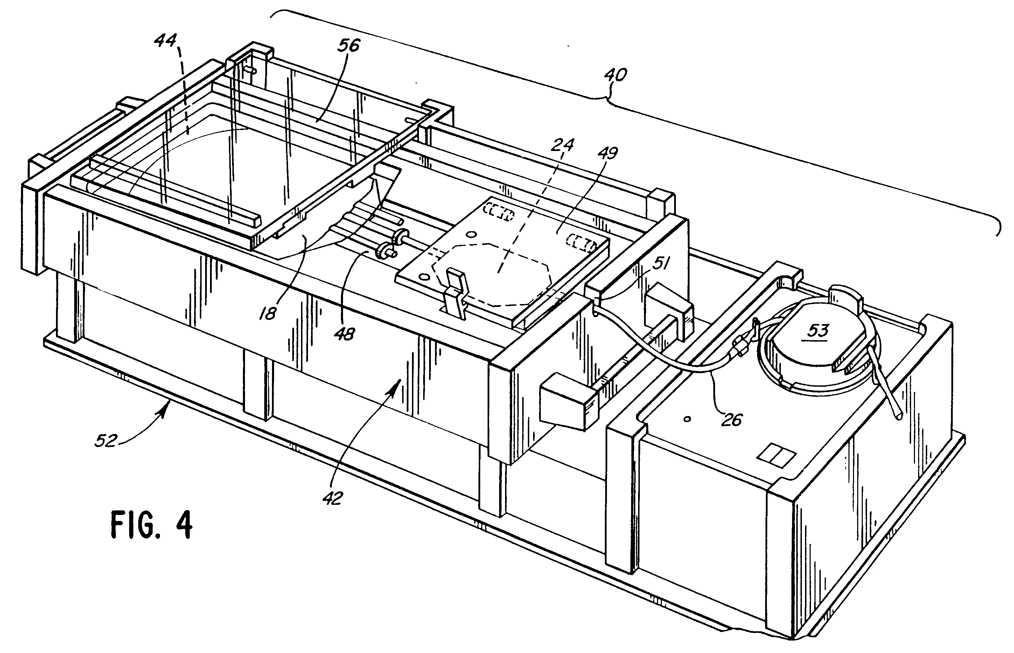

After priming the resulting interconnected bags system was installed into upper magnet tray 42 as shown particularly in Fig. 2, with bag 28 being placed in area 44, and container 24 being placed in area 46. The appropriate tubing 22 was stored in area 48, passing through channel 47, while tube 26 was installed in channel 51, and threaded through roller pump 53. The respective magnets 44, 46 were prechilled to about 4° C for continued cooling of the cells. Hinged covers 49, 56 were then closed, with the upper tray 42 installed on lower tray 52 (Fig. 4).

After about 5 minutes had elapsed, to allow complete bonding of the appropriate cells to the beads through the bonded CD4 antibodies, and to allow maximum capture of the resulting bead-cell conjugates at the inner surface of bag 18 adjacent magnet 44, roller pump 53 was actuated to pump at a rate of 10 ml. of fluid per minute into collection bag 32. Any bead-cell conjugates that escaped from bag 18 were subjected to the influence of secondary magnet 46 in bag 24, to be captured on the inner surface of chamber 24 adjacent that magnet, while cells which were not so bonded to the beads flowed out with fluids to collection bag 32.

After substantial emptying of bag 18 and container 24 of fluids, the bead-cell conjugate in bag 18 was resuspended in 100 ml. of physiological saline, while removing bag 18 and container 24 from their seating on the respective magnets, and closing flow through tubing 26 while so doing. Following this, bag 18 and container 24 were reinstalled in their positions on upper magnet tray 42, and the suspending saline solution withdrawn from bag 18 by roller pump 53, as before, through tubing 26 into collection bag 32, along with any remaining cells which were not bonded to a paramagnetic bead.

Then, bag 18 and container 24 were once again removed from the influence of the magnets, after closing off flow through tubing 26. The bead-cell conjugate in bag 18 and container 24 was resuspended in 100 ml. of physiological saline containing 25mM dithiothreitol to break the bond between cells and beads. Bag 18 and container 24 were placed on a rotator at 4° C for 20 minutes. Then, bag 18 and container 24 were connected to a 1 liter Fenwal PL269 tissue culture flask (symbolized in Fig. 1 as container 34). Following this, bag 18 and container 24 were reinstalled in upper tray 42, the hinged presser plates were closed, and the bag fluid contents were flushed into the tissue culture flask 34 while the paramagnetic bead particles remain trapped adjacent the magnets.

Thus, the free lymphocytes which are positive to the action of CD4 antibody, have been separated from other cells in the mixture, and are provided in isolated form in the tissue culture flask 34, or, if desired, a blood bag 34 as specifically shown in Fig. 1.

Residual cells in the separation set downstream from bag 24 are also flushed into flask or bag 34 with more physiological saline.

The cells in flask 34 may be collected by centrifugation and resuspended in 100 ml. RPMI 1640 tissue culture medium containing supplemental glutamine and placed in a carbon dioxide incubator for subsequent further study.

### EXAMPLE 2

By means of a process similar to that disclosed in Example 1, there may be removed from bone marrow cells generally, cells of the following types: B-lymphoma, neuroblastoma, breast cancer, or leukemia cells, when such cells express a tumor associated antigen on their surface recognizable by a biological attached to the bead particle surface. Thus, the process may be used or purifying bone marrow cells prior to autologous bone marrow transplant.

The bone marrow may be processed through a conventional cell separator to extract a mononuclear cell preparation therefrom. This mononuclear cell preparation may be incubated with a panel of mouse-derived monoclonal antibodies against the tumor cells, washed to remove excess antibody, and then incubated with paramagnetic beads as described in Example 1, coated with goat antimouse antibody to selectively bind the tumor cell to the bead. The tumor cell is then removed as in Example 1, using a magnetic separator of the type disclosed.

### EXAMPLE 3

As an alternative technique of the use of this invention, T-lymphocytes may be removed from the mononuclear cell preparation described in Example 2 before an allogenic transplant to prevent Graft vs. Host Disease. This may be used in the case where bone marrow grafts are provided for the treatment of cancer, or for reconstitution of the bone marrow after exposure to radiation apart from cancer treatment. In this case, the Mononuclear cell preparation from the bone marrow is treated with a mouse monoclonal antibody (for example, anti CD3) to tag the T-lymphocyte cells for binding by the goat antimouse antibody coated beads.

### EXAMPLE 4

The mononuclear cell preparation previously prepared from bone marrow as in Example 2 may also be treated in a manner similar to that described above, making use of a mouse monoclonal antibody bonded to the paramagnetic beads to allow the selection of pluripotent stems cells from the mononuclear cell preparation. The isolated stem cells can thus be used for bone marrow transplant. This approach could be used to provide for autologous bone marrow transplants which are substantially free of tumor cells, while suppressing Graft vs. Host Disease. Such isolated stem cells could also be used for gene therapy, in which genes are inserted into the stem cells prior to their implantation as a bone marrow transplant.

### EXAMPLE 5

By a process substantially similar to the process of Example 1, making use of an appropriate monoclonal antibody, liver hepatocytes or insulin secreting pancreatic beta cells may be isolated and thereafter cultured in an appropriate culture medium to expand the cells for use in organ transplantation.

### EXAMPLE 6

By a process similar to that of Example 1, making use of an appropriate monoclonal antibody, specific populations of cytotoxic T-lymphocytes may be selected, such as tetanus-toxoid primed lymphocyte cells. These cells can be separated by an analogous technique similar to that of Example 1, manipulated in vitro and transfused to effect targeting of the cytotoxic cells to specific B-lymphocytes which mediate autoimmune disease (for example, Myasthenia Gravis). In this way, the disease mediating cells could be destroyed in situ.

## Claims

1. An apparatus for the selective separation of a specific cell population from a heterogeneous cell mixture comprising a container (18) containing particle means (20) formed from a paramagnetic material, said particle means (20) having attached thereto a substance capable of binding to the specific cell population of said cell mixture; a cell flow path (16,22), with the container (18) positioned in the flow path (16,22) for receiving the cell mixture, such that in use said cell mixture intimately contacts, and is incubated with, said particle means within said container (18) to selectively bind said specific cell population of said cell mixture to said particle means (20), thereby creating a particle/cell conjugate; and first and second magnetic members (44,46), each positioned relative to said flow path, with said second magnetic member (46) being spaced downstream from said first magnetic member (16,22), to provide respective magnetic fields at fixed locations along said flow path to retain said particle/cell conjugate in said fixed locations and allow remaining, unbound portions of said cell mixture to be removed from said locations (44), characterised in that said second magnetic member (46) provides a shallower magnetic field at its associated location, but has a stronger magnetic holding force at its surface than the first magnetic member (44).

2. The apparatus of Claim 1, in which said container (18) is a flexible, collapsible container.

3. The apparatus of any preceding claim, comprising at least a second container (24) downstream of the first container (18), the second container (24) being flexible and collapsible.

4. The apparatus of claim 3 in which said second container (24) comprises inlet and outlet ports at opposed ends thereof, the cross sectional area of said inlet and outlet ports being no more than one-quarter of the cross sectional area of said second container (24).

5. The apparatus of Claim 4 in which said second container (24) is of hexagonal shape, said inlet and outlet ports being positioned at opposed corners thereof.

6. The apparatus of Claim 3, 4 or 5, in which said second magnetic member (46) is positioned adjacent means (42) carrying said second container (24), whereby at least one inner wall portion (59) of said second container (24) is within the magnetic field of said second magnetic member (46), said inner wall portion (59) being a said fixed location.

7. The apparatus of Claim 6 including flat-pressing means (49) to press said collapsible second container (24) flat while said inner wall portion (59) of said second container (24) is within the magnetic field of the second magnetic member (46) to provide a cell flow path thickness of 0.5 to 2.6 mm (0.02 to 0.1 inch) in said second container (24) to facilitate separation of said particle/cell conjugate from the remainder of said cell mixture.

8. The apparatus of any preceding claim, wherein the first magnetic member (44) is positioned adjacent means (42) carrying the first container, whereby an inner wall portion (57) of the first container (18) is within the magnetic field of said first magnetic member (44), said inner wall portion (57) of the first container (18) being a said fixed location.

9. The apparatus of any one of Claims 3 to 8 in which said second container (24) is sealingly and aseptically connected to the first container (18), said first magnet member (44) being positioned against said the first container (18) and said second magnet member (46) being positioned against said second container (24).

10. The apparatus of any preceding claim, comprising a collapsible receptacle (32) downstream of the first container (18).

11. The apparatus of Claim 10 wherein the first container (18) is aseptically connected to said receptacle (32), whereby the receptacle (32) is positioned to receive processed cells from the first container (18) without the need of forming a sterile connection therebetween.

12. The apparatus of Claim 10 or 11, wherein said second container (24) is sealingly and aseptically connected to and positioned between said first container (18) and said receptacle (32).

13. The apparatus of any preceding claim, comprising a fraction container for use with a cell mixture separating means (12), the fraction container being positioned upstream of the first container (18).

14. The apparatus of Claim 13, wherein said cell mixture is blood and said fraction container is a flexible, multiple-chamber insert means for a blood cell separation centrifuge, the first container (18) being aseptically connected to the insert means to enable a concentrated fraction of freshly collected blood cells to be aseptically transferred from said insert means to the first container (18) without need of forming a sterile connection therebetween.

15. The apparatus of any preceding claim wherein said first magnetic means (44) possesses a magnetic field having a reach substantially equivalent to the width of the first container (18).

16. The apparatus of Claim 15 wherein said first magnet (44) possesses a magnetic field having a reach of from about one-half an inch to about one inch (1.2 to 2.6 cm).

17. The apparatus of Claim 15 or 16 wherein said first magnetic member (44) possesses a magnetic field having a reach of from about one-half to about three quarters of an inch (1.2 to 1.9 cm).

18. The apparatus of any one of Claims 1 to 17, in which said paramagnetic particle means (20) carry on their surfaces a specific antibody to cells selected from the group consisting of B-lymphoma, neuroblastoma, breast cancer, leukaemia, T-lymphocytes, and pluripotent stem cells, or are coated with a specific antibody to cells selected from the group consisting of liver hepatocytes and insulin secreting pancreatic beta cells.

19. The apparatus Claim 18, in which said paramagnetic particle means (20) carry on their surfaces a specific antibody to a specific population of cytotoxic T-lymphocytes.

20. A method for the selective separation of a specific population of cells from a mixture of cells, the method comprising (a) mixing the cell mixture with paramagnetic particle means (20) in a container (18), the particle means (20) having attached thereto a substance capable of binding to the specific cell population of the mixture only, (b) exposing the container (18) to a magnetic field to immobilize said particle means (20) and the specific population of cells bound thereto, and (c) flowing unbound cells to first storage container means (32), whereby the first storage container means (32) receives cells not bound to said particle means (20), characterised by flowing cells from the first container (18) to a second container (24) at step (b), while exposing the first container to a first magnetic field, and exposing the second container (24) to a second magnetic field which is shallower than the first magnetic field and which has a stronger magnetic holding force at the surface of its associated magnet than the first magnetic field.

21. A method according to Claim 20, wherein the magnetic field reach of the first magnet (44) is substantially equivalent to the width of said first container (18).

22. A method according to Claim 20 or 21, wherein the cell mixture is a blood fraction, and further comprising the steps of centrifuging whole blood to separate out the fraction, collecting the blood fraction in the first container (18) and sealing said first container (18) from the centrifuge.

23. A method according to Claim 20, 21 or 22, further comprising the steps of positioning said first and second magnets (44,46) against said first and second containers (18,24) respectively in order to expose the containers (18,24) to the magnetic fields.

24. A method according to any one of Claims 20 to 23, further comprising stopping flow between said second container (24) and said first storage container means (32); removing said first and second containers (18,24) from the magnetic field of the respective magnet (44,46) and flowing the specific cell population, (20) from said first and second containers (18,24) into a second storage container means (34).

25. The method of Claim 24, wherein the cells are bound to the paramagnetic particle means (20) when they are flowed from the containers (18,24) into the second storage container means (34).

26. The method of Claim 24, further comprising disconnecting fluid communication between said second container (24) and the first storage container means (32), removing the binding connections between the cells of the specific population and the paramagnetic particle means (20) so that the specific cell population is no longer bound to the particle means (20), and flowing the cell population into said second storage container means (34) while the first and second containers (18,24) are under the influence of said magnetic fields, whereby said particle means (20) are prevented from flowing with said cells into the second storage container means (34), thereby separating the cells from the particle means.

27. The method of any one of Claims 20 to 26 in which said mixture of cells placed in the first container having paramegnetic particle means is a preparation of mononuclear cells previously separated from bone marrow.

28. The method of any one of Claims 20 to 27 when carried out using apparatus according to any one of Claims 1 to 19.

## Patentansprüche

1. Vorrichtung zum selektiven Trennen einer spezifischen Zellpopulation aus einem heterogenen Zellgemisch, wobei die Vorrichtung aufweist: einen Behälter (18), der Teilchen (20) enthält, die aus einem paramagnetischen Material gebildet sind, wobei an den Teilchen (20) eine Substanz haftet, die fähig ist, an die spezifische Zellpopulation des Zellgemischs zu binden; eine Zelldurchflußbahn (16, 22), wobei der Behälter (18) in der Durchflußbahn (16, 22) positioniert ist, um das Zellgemisch aufzunehmen, so daß im Gebrauch das Zellgemisch mit den Teilchen in dem Behälter (18) in innigen Kontakt gelangt und damit inkubiert wird, um die spezifische Zellpopulation des Zellgemischs selektiv an die Teilchen (20) zu binden, wodurch ein Teilchen/Zell-Konjugat gebildet wird; und ein erstes und ein zweites magnetisches Element (44, 46), die jeweils relativ zu der Durchflußbahn positioniert sind, wobei das zweite magnetische Element (46) an der Abstromseite von dem ersten magnetischen Element (16, 22) beabstandet ist, um an unveränderlichen Stellen entlang der Durchflußbahn jeweilige Magnetfelder zu bilden, um das Teilchen/Zell-Konjugat an den unveränderlichen Stellen festzuhalten und zuzulassen, daß verbleibende, ungebundene Teile des Zellgemischs von diesen Stellen (44) entfernt werden, dadurch gekennzeichnet, daß das zweite magnetische Element (46) an seiner zugeordneten Stelle ein seichteres Magnetfeld bildet, aber an seiner Oberfläche eine größere magnetische Haltekraft als das erste magnetische Element (44) hat.

2. Vorrichtung nach Anspruch 1, wobei der Behälter (18) ein flexibler, kollabierbarer Behälter ist.

3. Vorrichtung nach einem der vorhergehenden Ansprüche, die mindestens einen zweiten Behälter (24) an der Abstromseite des ersten Behälters (18) aufweist, wobei der zweite Behälter (24) flexibel und kollabierbar ist.

4. Vorrichtung nach Anspruch 3, wobei der zweite Behälter (24) an seinen entgegengesetzten Enden eine Einlaß- und eine Auslaßöffnung aufweist, wobei die Querschnittsfläche der Einlaß- und der Auslaßöffnung nicht mehr als ein Viertel der Querschnittsfläche des zweiten Behälters (24) ist.

5. Vorrichtung nach Anspruch 4, wobei der zweite Behälter (24) Sechseckgestalt hat, wobei die Einlaß- und die Auslaßöffnung an entgegengesetzten Ecken davon positioniert sind.

6. Vorrichtung nach Anspruch 3, 4 oder 5, wobei das zweite magnetische Element (46) angrenzend an eine Einrichtung (42), die den zweiten Behälter (24) trägt, positioniert ist, wodurch mindestens ein Innenwandbereich (59) des zweiten Behälters (24) innerhalb des Magnetfelds des zweiten magnetischen Elements (46) liegt, wobei der Innenwandbereich (59) eine genannte unveränderliche Stelle ist.

7. Vorrichtung nach Anspruch 6, die eine Flachdrückeinrichtung (49) aufweist, um den kollabierbaren zweiten Behälter (24) flachzudrücken, während gleichzeitig der Innenwandbereich (59) des zweiten Behälters (24) innerhalb des Magnetfelds des zweiten magnetischen Elements (46) liegt, um eine Zelldurchflußbahndicke von 0,5-2,6 mm (0,02-0,1") in dem zweiten Behälter (24) zu bilden, um das Trennen des Teilchen/Zell-Konjugats von dem restlichen Zellgemisch zu erleichtern.

8. Vorrichtung nach einem der vorhergehenden Ansprüche, wobei das erste magnetische Element (44) angrenzend an eine Einrichtung (42), die den ersten Behälter trägt, positioniert ist, wodurch ein Innenwandbereich (57) des ersten Behälters (18) innerhalb des Magnetfelds des ersten magnetischen Elements (44) liegt, wobei der Innenwandbereich (57) des ersten Behälters (18) eine genannte unveränderliche Stelle ist.

9. Vorrichtung nach einem der Ansprüche 3 bis 8, wobei der zweite Behälter (24) mit dem ersten Behälter (18) abdichtend und aseptisch verbunden ist, wobei das erste magnetische Element (44) an dem ersten Behälter (18) anliegend positioniert ist und das zweite magnetische Element (46) an dem zweiten Behälter (24) anliegend positioniert ist.

10. Vorrichtung nach einem der vorhergehenden Ansprüche, die einen kollabierbaren Aufnahmebehälter (32) an der Abstromseite des ersten Behälters (18) aufweist.

11. Vorrichtung nach Anspruch 10, wobei der erste Behälter (18) mit dem Aufnahmebehälter (32) aseptisch verbunden ist, wodurch der Aufnahmebehälter (32) positioniert ist, um verarbeitete Zellen aus dem ersten Behälter (18) aufzunehmen, ohne daß dazwischen eine sterile Verbindung gebildet werden muß.

12. Vorrichtung nach Anspruch 10 oder 11, wobei der zweite Behälter (24) mit dem ersten Behälter (18) und dem Aufnahmebehälter (32) abdichtend und aseptisch verbunden und dazwischen positioniert ist.

13. Vorrichtung nach einem der vorhergehenden Ansprüche, die einen Fraktionsbehälter zur Verwendung mit einer Zellgemisch-Trenneinrichtung (12) aufweist, wobei der Fraktionsbehälter an der Aufstromseite des ersten Behälters (18) positioniert ist.

14. Vorrichtung nach Anspruch 13, wobei das Zellgemisch Blut ist und der Fraktionsbehälter eine flexible Mehrkammer-Einsatzeinrichtung für eine Blutzellen-Trennzentrifuge ist, wobei der erste Behälter (18) mit der Einsatzeinrichtung aseptisch verbunden ist, um zu ermöglichen, daß eine konzentrierte Fraktion von frisch gesammelten Blutzellen aus der Einsatzeinrichtung in den ersten Behälter (18) aseptisch überführt wird, ohne daß dazwischen eine sterile Verbindung gebildet werden muß.

15. Vorrichtung nach einem der vorhergehenden Ansprüche, wobei die erste magnetische Einrichtung (44) ein Magnetfeld hat, dessen Reichweite der Breite des ersten Behälters (18) im wesentlichen äquivalent ist.

16. Vorrichtung nach Anspruch 15, wobei der erste Magnet (44) ein Magnetfeld hat, das eine Reichweite von ca. 1,2 bis ca. 2,6 cm (ca. 0,5 bis ca. 1") hat.

17. Vorrichtung nach Anspruch 15 oder 16, wobei das erste magnetische Element (44) ein Magnetfeld hat, das eine Reichweite von ca. 1,2 bis ca. 1,9 cm (ca. 0,5 bis ca. 0,75") hat.

18. Vorrichtung nach einem der Ansprüche 1 bis 17, wobei die paramagnetischen Teilchen (20) an ihren Oberflächen einen spezifischen Antikörper für Zellen tragen, die aus der Gruppe ausgewählt sind, die aus B-Lymphom-, Neuroblastom-, Brustkrebs-, Leukämie-, T-Lymphozyten- und pluripotenten Stammzellen besteht, oder mit einem spezifischen Antikörper für Zellen beschichtet sind, die aus der Gruppe ausgewählt sind, die aus Hepatozyten und Insulin sekretierenden pankreatitischen Betazellen besteht.

19. Vorrichtung nach Anspruch 18, wobei die paramagnetischen Teilchen (20) an ihren Oberflächen einen spezifischen Antikörper für eine spezifische Population von zytotoxischen T-Lymphozyten tragen.

20. Verfahren zum selektiven Trennen einer spezifischen Zellpopulation aus einem Zellgemisch, wobei das Verfahren die folgenden Schritte aufweist: (a) Vermischen des Zellgemischs mit paramagnetischen Teilchen (20) in einem Behälter (18), wobei an den Teilchen (20) eine Substanz haftet, die fähig ist, nur an die spezifische Zellpopulation des Gemischs zu binden; (b) Aussetzen des Behälters (18) einem Magnetfeld, um die Teilchen (20) und die daran gebundene spezifische Zellpopulation zu immobilisieren, und (c) Fließenlassen nicht gebundener Zellen zu einer ersten Speicherbehältereinrichtung (32), wobei die erste Speicherbehältereinrichtung (32) Zellen aufnimmt, die nicht an die Teilchen (20) gebunden sind, gekennzeichnet durch Fließenlassen von Zellen aus dem ersten Behälter (18) in einen zweiten Behälter (24) in Schritt (b), während gleichzeitig der erste Behälter einem ersten Magnetfeld ausgesetzt wird, und Aussetzen des zweiten Behälters (24) einem zweiten Magnetfeld, das seichter als das erste Magnetfeld ist und an der Oberfläche seines zugeordneten Magneten eine größere magnetische Haltekraft als das erste Magnetfeld hat.

21. Verfahren nach Anspruch 20, wobei die Reichweite des Magnetfelds des ersten Magneten (44) der Breite des ersten Behälters (18) im wesentlichen äquivalent ist.

22. Verfahren nach Anspruch 20 oder 21, wobei das Zellgemisch eine Blutfraktion ist und das Verfahren ferner die folgenden Schritte aufweist: Zentrifugieren von Vollblut, um die Fraktion herauszutrennen, Sammeln der Blutfraktion in dem ersten Behälter (18) und dichtes Verschließen des ersten Behälters (18) gegenüber der Zentrifuge.

23. Verfahren nach Anspruch 20, 21 oder 22, das ferner die folgenden Schritte aufweist: Positionieren des ersten und des zweiten Magneten (44, 46) anliegend an den ersten bzw. den zweiten Behälter (18, 24), um die Behälter (18, 24) den Magnetfeldern auszusetzen.

24. Verfahren nach einem der Ansprüche 20 bis 23, das ferner die folgenden Schritte aufweist: Unterbrechen des Durchflusses zwischen dem zweiten Behälter (24) und der ersten Speicherbehältereinrichtung (32); Entfernen des ersten und des zweiten Behälters (18, 24) aus dem Magnetfeld des jeweiligen Magneten (44, 46) und Fließenlassen der spezifischen Zellpopulation aus dem ersten und dem zweiten Behälter (18, 24) in eine zweite Speicherbehältereinrichtung (34).

25. Verfahren nach Anspruch 24, wobei die Zellen an die paramagnetischen Teilchen (20) gebunden werden, wenn man sie aus den Behältern (18, 24) in die zweite Speicherbehältereinrichtung (34) fließen läßt.

26. Verfahren nach Anspruch 24, das ferner die folgenden Schritte aufweist: Unterbrechen der Fluidverbindung zwischen dem zweiten Behälter (24) und der ersten Speicherbehältereinrichtung (32), Entfernen der Bindungs-Verbindungen zwischen den Zellen der spezifischen Population und den paramagnetischen Teilchen (20), so daß die spezifische Zellpopulation nicht mehr an die Teilchen (20) gebunden ist, und Fließenlassen der Zellpopulation in die zweite Speicherbehältereinrichtung (34), während gleichzeitig der erste und der zweite Behälter (18, 24) unter dem Einfluß der Magnetfelder stehen, wodurch verhindert wird, daß die Teilchen (20) mit den Zellen in die zweite Speicherbehältereinrichtung (34) fließen, um dadurch die Zellen von den Teilchen zu trennen.

27. Verfahren nach einem der Ansprüche 20 bis 26, wobei das in den ersten Behälter mit paramagnetischen Teilchen eingebrachte Zellgemisch ein Präparat aus zuvor aus Knochenmark getrennten einkernigen Zellen ist.

28. Verfahren nach einem der Ansprüche 20 bis 27, bei dessen Durchführung die Vorrichtung nach einem der Ansprüche 1 bis 19 verwendet wird.

## Revendications

1. Installation pour la séparation sélective d'une population spécifique de cellules d'un mélange hétérogène de cellules comprenant un conteneur (18) contenant des éléments de particules (20) formés à partir d'un matériau paramagnétique, une substance capable de se lier à la population spécifique de cellules dudit mélange de cellules étant reliée auxdits éléments de particules (20) ; un trajet d'écoulement de cellules (16, 22), le conteneur (18) étant disposé sur le trajet d'écoulement (16, 22) pour recevoir le mélange de cellules, de sorte qu'en fonctionnement, ledit mélange de cellules est en contact étroit et est mis à incuber avec lesdits éléments de particules à l'intérieur dudit conteneur (18) afin de lier de façon sélective ladite population spécifique de cellules dudit mélange de cellules auxdits éléments de particules (20), créant ainsi un conjugué particules/cellules ; et des premier et deuxième éléments magnétiques (44, 46), disposés chacun par rapport audit trajet d'écoulement, ledit deuxième élément magnétique (46) étant espacé en aval dudit premier élément magnétique (44), pour créer des champs magnétiques respectifs à des emplacements fixés le long dudit trajet d'écoulement afin de retenir ledit conjugué particules/cellules dans ledit emplacement fixé et permettre aux parties restantes non liées dudit mélange de cellules d'être éliminées desdits emplacements (44), caractérisée en ce que ledit deuxième élément magnétique (46) crée un champ magnétique moins profond à son emplacement associé, mais présente une force de maintien magnétique plus importante à sa surface que le premier élément magnétique (44).

2. Installation selon la revendication 1, dans laquelle ledit conteneur (18) est un conteneur souple écrasable.

3. Installation selon l'une quelconque des revendications précédentes, comprenant au moins un deuxième conteneur (24) en aval du premier conteneur (18), le deuxième conteneur (24) étant souple et écrasable.

4. Installation selon la revendication 3, dans laquelle ledit deuxième conteneur (24) comprend des orifices d'entrée et de sortie à ses extrémités opposées, l'aire de la section transversale desdits orifices d'entrée et de sortie ne dépassant pas un quart de l'aire de la section transversale dudit deuxième conteneur (24).

5. Installation selon la revendication 4, dans laquelle ledit deuxième conteneur (24) est de forme hexagonale, lesdits orifices d'entrée et de sortie étant placés à des angles opposés de celui-ci.

6. Installation selon la revendication 3, 4, ou 5, dans laquelle ledit deuxième élément magnétique (46) est placé près de moyens (42) portant ledit deuxième conteneur (24), au moins une partie de paroi intérieure (59) dudit deuxième conteneur (24) étant à l'intérieur du champ magnétique dudit deuxième élément magnétique (46), ladite partie de paroi intérieure (59) étant audit emplacement fixé.

7. Installation selon la revendication 6, comprenant des moyens de presse à plateau (49) pour aplatir ledit deuxième conteneur écrasable (24) pendant que ladite partie de paroi intérieure (59) dudit deuxième conteneur (24) est à l'intérieur du champ magnétique du deuxième élément magnétique (46) pour fournir une épaisseur de trajet d'écoulement de cellules de 0,5 à 2,6 mm (0,02 à 0,1 pouce) dans ledit deuxième conteneur (24) pour faciliter la séparation dudit conjugué particules/cellules du reste dudit mélange de cellules.

8. Installation selon l'une quelconque des revendications précédentes, dans laquelle le premier élément magnétique (44) est placé près de moyens (42) portant le premier conteneur (24), une partie de paroi intérieure (57) du premier conteneur (18) étant à l'intérieur du champ magnétique dudit premier élément magnétique (44), ladite partie de paroi intérieure (57) du premier conteneur (18) étant audit emplacement fixé.

9. Installation selon l'une quelconque des revendications 3 à 8, dans laquelle ledit deuxième conteneur (24) est relié de façon étanche et aseptique au premier conteneur (18), ledit premier élément magnétique (44) étant placé contre ledit premier conteneur (18) et ledit deuxième élément magnétique (46) étant placé contre ledit deuxième conteneur (24).

10. Installation selon l'une quelconque des revendications précédentes, comprenant un récipient écrasable (32) en aval du premier conteneur (18).

11. Installation selon la revendication 10, dans laquelle le premier conteneur (18) est relié de façon aseptique audit récipient (32), le récipient (32) étant placé pour recevoir des cellules traitées provenant du premier conteneur (18) sans nécessité de former une connexion stérile entre eux.

12. Installation selon la revendication 10 ou 11, dans laquelle ledit deuxième conteneur (24) est relié de façon étanche et aseptique audit premier conteneur (18) et audit récipient (32) et est placé entre eux.

13. Installation selon l'une quelconque des revendications précédentes, comprenant un conteneur de fraction à utiliser avec des moyens de séparation de mélange de cellules (12), le conteneur de fraction étant placé en amont du premier conteneur (18).

14. Installation selon la revendication 13, dans laquelle ledit mélange est du sang et ledit conteneur de fraction est un moyen souple à insert à chambres multiples pour une centrifugeuse de séparation de cellules sanguines, le premier conteneur (18) étant relié de façon aseptique au moyen souple à insert pour permettre à une fraction concentrée de cellules sanguines fraîchement recueillies d'être transférée de façon aseptique dudit moyen à insert au premier conteneur (18) sans nécessité de former une connexion stérile entre eux.

15. Installation selon l'une quelconque des revendications précédentes, dans laquelle ledit premier élément magnétique (44) possède un champ magnétique présentant une étendue sensiblement équivalente à la largeur du premier conteneur (18).

16. Installation selon la revendication 15, dans laquelle ledit premier élément magnétique (44) possède un champ magnétique présentant une étendue d'environ un demi-pouce à environ un pouce (1,2 à 2,6 cm).

17. Installation selon la revendication 15 ou 16, dans laquelle ledit premier élément magnétique (44) possède un champ magnétique présentant une portée d'environ un demi à environ trois quarts d'un pouce (1,2 à 1,9 cm).

18. Installation selon l'une quelconque des revendications 1 à 17, dans laquelle lesdits éléments de particules paramagnétiques (20) portent sur leurs surfaces un anticorps spécifique des cellules choisies parmi le groupe constitué des B-lymphome, neuroblastome, cancer du sein, leucémie, T-lymphocytes, et cellules tigées pluripotentes, ou sont revêtus d'un anticorps spécifique de cellules choisies parmi le groupe constitué des hépatocytes et des cellules pancréatiques bêta sécrétant de l'insuline;

19. Installation selon la revendication 18, dans laquelle lesdits éléments paramagnétiques (20) portent sur leurs surfaces un anticorps spécifique d'une population spécifique de T-lymphocytes cytotoxiques.

20. Procédé pour la séparation sélective d'une population spécifique de cellules d'un mélange de cellules, le procédé comprenant (a) le mélange du mélange de cellules avec les éléments de particules paramagnétiques (20) dans un conteneur (18), une substance capable de se lier uniquement à la population de cellules spécifique du mélange étant reliée aux éléments de particules (20), (b) l'exposition du conteneur (18) à un champ magnétique pour immobiliser lesdits éléments de particules (20) et la population spécifique de cellules qui y est liée, et (c) l'écoulement des cellules non liées vers le premier moyen de conteneur de stockage (32), moyennant quoi le premier moyen de conteneur de stockage (32) reçoit des cellules non liées auxdits éléments de particules (20), caractérisé par l'écoulement des cellules du premier conteneur (18) vers un deuxième conteneur (24) à l'étape (b) tout en exposant le premier conteneur à un premier champ magnétique, et l'exposition du deuxième conteneur (24) à un deuxième champ magnétique qui est plus superficiel que le premier champ magnétique et qui présente une force de maintien plus importante à la surface de son élément magnétique associé que le premier champ magnétique.

21. Procédé selon la revendication 20, dans lequel l'étendue du champ magnétique du premier élément magnétique (44) est sensiblement équivalente à la largeur dudit premier conteneur (18).

22. Procédé selon la revendication 20 ou 21, dans lequel le mélange de cellules est une fraction sanguine, et comprenant en outre les étapes consistant à centrifuger la totalité du sang pour extraire la fraction, recueillir la fraction sanguine dans le premier conteneur (18) et isoler hermétiquement ledit premier conteneur (18) de la centrifugeuse.

23. Procédé selon la revendication 20, 21 ou 22, comprenant en outre les étapes consistant à placer lesdits premier et deuxième éléments magnétiques (44, 46) contre lesdits premier et deuxième conteneurs (18, 24) respectivement dans le but d'exposer les conteneurs (18, 24) aux champs magnétiques.

24. Procédé selon l'une quelconque des revendications 20 à 23, comprenant en outre l'arrêt de l'écoulement entre ledit deuxième conteneur (24) et ledit premier moyen de conteneur de stockage (32) ; le retrait desdits premier et deuxième conteneurs (18, 24) du champ magnétique de l'élément magnétique respectif (44, 46) et l'écoulement de la population spécifique de cellules (20) desdits premier et deuxième conteneurs (18, 24) dans un deuxième moyen de conteneur de stockage (34).

25. Procédé selon la revendication 24, dans lequel les cellules sont liées aux éléments de particules paramagnétiques (20) quand ils s'écoulent des conteneurs (18, 24) dans le deuxième moyen de conteneur de stockage (34).

26. Procédé selon la revendication 24, comprenant en outre la coupure de la communication de fluide entre ledit deuxième conteneur (24) et le premier moyen de conteneur de stockage (32), le retrait des connexions de liaison entre les cellules de la population spécifique et les éléments de particules paramagnétiques (20) de sorte que la population spécifique de cellules n'est plus liée aux éléments de particules (20), et l'écoulement de la population de cellules dans ledit deuxième moyen de conteneur de stockage (34) pendant que les premier et deuxième conteneurs (18, 24) sont sous l'influence desdits champs magnétiques, moyennant quoi lesdits éléments de particules (20) sont empêchés de s'écouler avec lesdites cellules dans le deuxième moyen de conteneur de stockage (34), séparant ainsi les cellules des éléments de particules.

27. Procédé selon l'une quelconque des revendications 20 à 26, dans lequel ledit mélange de cellules placé dans le premier conteneur comprenant des éléments de particules paramagnétiques est une préparation de cellules mononucléaires préalablement séparées de la moelle osseuse.

28. Procédé selon l'une quelconque des revendications 20 à 27, quand il est mis en oeuvre en utilisant l'installation selon l'une quelconque des revendications 1 à 19.
